# EUROPÄISCHE PATENTSCHRIFT

(11) **EP 0 914 080 B1**
(45) Veröffentlichungstag und Bekanntmachung des Hinweises auf die Patenterteilung: **29.08.2001**
(21) Anmeldenummer: 97922961.4
(22) Anmeldetag: 05.05.1997
(51) Int. Cl.: A61K 7/13

(54) **MITTEL UND VERFAHREN ZUM FÄRBEN UND TÖNEN KERATINISCHER FASERN**
AGENT AND PROCESS FOR DYEING AND TINTING KERATINOUS FIBRES
AGENT ET PROCEDE POUR COLORER ET TEINTER DES FIBRES KERATINIQUES

(30) Priorität: 13.05.1996 DE 19619071
(43) Veröffentlichungstag der Anmeldung: 12.05.1999
(73) Patentinhaber: Henkel Kommanditgesellschaft auf Aktien, 40589 Düsseldorf-Holthausen (DE)
(72) Erfinder: HURSCHMANN, Brigitta, D-42855 Remscheid (DE); HOLLENBERG, Detlef, D-40699 Erkrath (DE)
(74) Vertreter: Foitzik, Joachim Kurt
(86) Internationale Anmeldenummer: EP9702280
(87) Internationale Veröffentlichungsnummer: WO9742932

(56) Entgegenhaltungen:
- EP-A- 0 545 257
- US-A- 5 042 988
- CHEMICAL ABSTRACTS, vol. 124, no. 18, 29.April 1996 Columbus, Ohio, US; abstract no. 241753g, Y. OGITA ET AL: "Hair dye compositions containing coloring-delaying agents" XP002037637 & JP 08 012 539 A (DAIICHI SEIYAKU) 16.Januar 1996

## Beschreibung

Die Erfindung betrifft Mittel zum Färben und Tönen keratinischer Fasern, insbesondere menschlicher Haare, mit einem speziellen Wirkstoffkomplex.

Zubereitungen zum Tönen und Färben von Haaren sind ein wichtiger Typ von kosmetischen Mitteln. Sie können dazu dienen, die natürliche Haarfarbe gemäß den Wünschen der entsprechenden Person leicht oder stärker zu nuancieren, eine gänzlich andere Haarfarbe zu erzielen oder unerwünschte Haartöne, wie beispielsweise Grautöne, zu überdecken. Übliche Haarfärbemittel werden, je nach gewünschter Farbe bzw. Dauerhaftigkeit der Färbung, entweder auf Basis von Oxidationsfarbstoffen oder auf Basis von direktziehenden Farbstoffen formuliert. Häufig werden auch Kombinationen von Oxidationsfarbstoffen und direktziehenden Farbstoffen zur Erzielung spezieller Nuancen eingesetzt.

Färbemittel auf Basis von Oxidationsfarbstoffen führen zu brillanten und dauerhaften Farbtönen. Sie bedingen allerdings den Einsatz starker Oxidationsmittel wie beispielsweise Wasserstoffperoxid-Lösungen. Dies führt häufig zu Schädigungen des zu färbenden Haares, denen mit entsprechenden Pflegeprodukten entgegengewirkt werden muß. Außerdem können Kontakte der Haut mit diesen Färbemitteln bei sehr empfindlichen Personen zu unerwünschten Reaktionen führen.
Färbemittel auf Basis direktziehender Farbstoffe kommen ohne Oxidationsmittel aus und können besser bei pH-Werten in der Gegend des Neutralpunktes formuliert werden. Ein wesentlicher Nachteil der Färbemittel auf Basis direktziehender Farbstoffe ist aber die geringe Waschechtheit der gefärbten Haare. Entsprechend können insbesondere das Aufziehvermögen der Farbstoffmoleküle auf das Haar sowie weiterhin der Glanz der gefärbten Haare in vielen Fällen nicht voll befriedigen.

Es besteht daher weiterhin das Bedürfnis nach Färbemitteln auf Basis direktziehender Farbstoffe, die sich durch ein verbessertes Aufziehvermögen der Farbstoffinoleküle auf das Haar und/oder einen verbesserten Glanz des gefärbten Haares auszeichnen.

Es wurde nun gefunden, daß mit einer Wirkstoffkombination aus Isoascorbinsäure und speziellen Aminocarbonsäuren den genannten Nachteilen in überraschend hohem Maße abgeholfen und insbesondere das Aufziehvermögen der Farbstoffmoleküle und der Glanz der Haare signifikant verbessert werden kann.

Gegenstand der Erfindung sind somit Mittel zum Färben oder Tönen keratinischer Fasern, insbesondere menschlichen Haaren, dadurch gekennzeichnet, daß es neben einem direktziehenden Farbstoff eine Wirkstoffkombination, bestehend aus
- einer Aminopolycarbonsäure oder deren Salz mit einem physiologisch verträglichen Kation,
- Isoascorbinsäure oder deren Salz mit einem physiologisch verträglichem Kation und
- eine gesättigte Carbonsäure mit 10-18 Kohlenstoffatomen enthält.

Unter keratinischen Fasern sind erfindungsgemäß Pelze, Wolle, Federn und insbesondere menschliche Haare zu verstehen. Obwohl die erfindungsgemäßen Färbemittel in erster Linie zum Färben von keratinischen Fasern geeignet sind, steht prinzipiell einer Verwendung auch auf anderen Gebieten nichts entgegen.

Die erfindungsgemäße Wirkstoffkombination besteht aus zwei zwingenden Bestandteilen

Der erste Bestandteil ist eine Aminopolycarbonsäure. Unter Aminopolycarbonsäure ist eine Verbindung zu verstehen, die mindestens eine, gegebenenfalls substituierte, Aminogruppe und mindestens 2 Carbonsäuregruppen aufweist. Als besonders geeignet haben sich solche Aminopolycarbonsäuren erwiesen, die zur Ausbildung von Chelatkomplexen befähig sind.

Bevorzugte erfindungsgemäß eingesetzte Aminopolycarbonsäuren sind Nitrilotriessigsäure, Ethylendiamintetraessigsäure, Diethylentriaminopentaessigsäure und N-Hydroxyethylethylendiaminotriessigsäure. Unter diesen Verbindungen ist die Ethylendiamintetraessigsäure besonders bevorzugt.

Die Aminopolycarbonsäuren können den erfindungsgemäßen Mitteln als freie Säuren zugegeben werden. Es ist aber auch möglich, die Aminopolycarbonsäuren in Form von Salzen mit physiologisch verträglichen Kationen zuzugeben. Solche Kationen sind beispielsweise Alkali-, Erdalkali-, Aluminium-, Ammonium- sowie Mono-, Di- und Trialkanolammoniumionen. Alkalimetallionen, insbesondere Natriumionen, sind bevorzugte Ionen.

Als hervorragend geeignete Komponente der erfindungsgemäßen Wirkstoffkombination hat sich das Di- und insbesondere das Tetranatriumsalz der Ethylendiamintetraessigsäure erwiesen.
Die erfindungsgemäßen Mittel enthalten die Aminopolycarbonsäure oder ihr Salz bevorzugt in einer Menge von 0,01 bis 1 Gew.-%, bezogen auf das gesamte Mittel. Mengen von 0,05 bis 0,5 Gew.-% sind besonders bevorzugt.

Als zweite zwingende Komponente enthält die erfindungsgemäße Wirkstoffkombination Isoascorbinsäure oder deren Salz mit einem physiologisch verträglichen Kation.

Die erfindungsgemäßen Mittel enthalten die Isoascorbinsäure bevorzugt in einer Menge von 0,01 - 1 Gew.-%, bezogen auf das gesamte Mittel. Mengen von 0,05 bis 0,5 Gew.-% sind besonders bevorzugt.

Die erfindungsgemäßen Mittel zeigten besonders gute Ergebnisse, wenn die Mengenverhältnisse (= Massenverhältnisse) von Aminopolycarbonsäure und Isoascorbinsäure zwischen 2:1 und 1:2 betrugen.

Weiterhin enthalten die erfindungsgemäßen Mittel mindestens einen direktziehenden Farbstoff. Dieser wird üblicherweise aus der Gruppe der Nitrophenylendiamine, Nitroaminophenole, Anthrachinone oder Indophenole ausgewählt. Entsprechende Verbindungen sind beispielsweise die unter den internationalen Bezeichnungen bzw. Handelsnamen HC Yellow 2, HC Yellow 4, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16, Pikraminsäure und Rodol 9 R bekannten Verbindungen sowie 4-Amino-2-nitro-diphenylamin-2'-carbonsäure, 6-Nitro-1,2,3,4-tetrahydrochinoxalin, (N-2,3-Dihydroxypropyl-2-nitro-4-trifluormethyl)aminobenzol und 4-N-Ethyl-1,4-bis(2'-hydroxyethylamino)-2-nitrobenzol-hydrochlorid.
Unter direktziehenden Farbstoffen werden erfindungsgemäß auch Naturfarbstoffe, wie beispielsweise Henna rot, Henna schwarz, Henna neutral, Kamillenblüte, Sandelholz, schwarzer Tee, Faulbaumrinde, Salbei, Blauholz, Krappwurzel, Catechu, Sedre und Alkannawurzel verstanden.

Die direktziehenden Farbstoffe sind in den erfindungsgemäßen Mitteln bevorzugt in Mengen von jeweils 0,01 bis 20 Gew.-%, bezogen auf das gesamte Mittel, enthalten.

Weiterhin können die erfindungsgemäßen Mittel Oxidationsfarbstoffvorprodukte, sogenannte Entwicklerkomponenten und Kupplerkomponenten, enthalten. Die Entwicklerkomponenten bilden unter dem Einfluß von Oxidationsmitteln oder von Luftsauerstoff, gegebenenfalls mit Hilfe spezieller Enzyme, untereinander oder unter Kupplung mit einer oder mehreren Kupplerkomponenten die eigentlichen Farbstoffe aus.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt. Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 4-Amino-2-(2-hydroxyethoxy)-phenol, 4-Hydroxy-2,5,6-triaminopyrimidin, 2-Hydroxy-4,5,6-triaminopyrimidin, 2,4-Dihydroxy-5,6-diaminopyrimidin und 2,5,6-Triaminohydroxypyrimidin.
Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlor-resorcin, 4-Chlor-resorcin, 2-Chlor-6-methyl-3-aminophenol, 2-Amino-4-hydroxypyridin, 2-Methylresorcin und 5-Methylresorcin.

Erfindungsgemäß bevorzugt sind solche Entwickler- und Kupplerkomponenten, die für die Ausbildung der Färbungen keine Oxidationsmittel außer Luftsauerstoff benötigen.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die erfindungsgemäßen Haarfärbemittel enthalten sowohl die Entwicklerkomponenten als auch die Kupplerkomponenten bevorzugt in einer Menge von 0,005 bis 20° Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1 : 0,5 bis 1 : 2 enthalten sein können.

Bezüglich weiterer üblicher Farbstoffkomponenten wird ausdrücklich auf die Colipa-Liste, herausgegeben vom Industrieverband Körperpflege und Waschmittel, Frankfurt, Bezug genommen.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die durch die erfindungsgemäßen Mittel erhaltenen Effekte konnten noch gesteigert werden, wenn diese Mittel neben den beiden zwingenden Komponenten der Wirkstoffkombination weiterhin eine Carbonsäure mit 8 bis 18 Kohlenstoffatomen enthielt.

Prinzipiell können, soweit es die Konsistenz der gewünschten Formulierung zuläßt, sowohl gesättigte als auch ein- oder mehrfach ungesättigte, lineare oder verzweigte Carbonsäure eingesetzt werden. Als besonders effektiv haben sich aber die gesättigten Carbonsäuren mit 10 bis 18 Kohlenstoffatomen erwiesen. Myristinsäure, Palmitinsäure und insbesondere Laurinsäure sind daher besonders bevorzugte weitere Komponenten der erfindungsgemäßen Mittel.

Eine weitere Komponente, die sich vorteilhaft auf die Eigenschaften der erfindungsgemäßen Mittel auswirkt, sind oligomere und polymere Polyole. Bevorzugte Verbindungen sind Polyethylenglykole, Polypropylenglykole und Polyglycerine. Polyethylenglykole sind unter diesen Polyolen besonders bevorzugt. Diese Polyole haben bevorzugt mit Molmasse von 500 bis 3000 Dalton; Molmassen zwischen 1000 und 2000 Dalton, insbesondere um etwa 1500 Dalton, sind besonders bevorzugt.

Zur Herstellung der erfindungsgemäßen Färbemittel werden die Oxidationsfarbstoffvorprodukte in einen geeigneten wasserhaltigen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammoniumsowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil® -Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid® S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Dialkylammoniummethosulfate und Methyl-hydroxyalkyldialkoyloxyalkyl-ammonium-methosulfate.

Ein Beispiel für ein als kationisches Tensid einsetzbares quaternäres Zuckerderivat stellt das Handelsprodukt Glucquat® 100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Tenside liegen in den erfindungsgemäßen Mitteln bevorzugt in Mengen von 1 - 10, insbesondere etwa 6 - 10 Gew.-%, jeweils bezogen auf das gesamte Mittel, vor, wenn es sich bei dem erfindungsgemäßen Mittel um ein Färbeshampoo handelt. Bei Färbelotionen oder Färbegelen sind dagegen Mengen von 1 - 5, insbesondere 2 - 4 Gew.-%, jeweils bezogen auf das gesamte Mittel, bevorzugt.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Farbstoffe zum Einfärben der Zubereitungen,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Gegenstand der Anmeldung ist auch die Verwendung eines erfindungsgemäßen Mittels zum Färben und Tönen von keratinischen Fasern, insbesondere menschlichen Haaren.

Wenngleich es möglich ist, die erfindungsgemäßen Mittel so zu formulieren, daß sie auf dem Haar verbleiben können, so ist es doch bevorzugt solche Mittel einzusetzen, die wieder aus dem Haar ausgespült werden.

Gegenstand der Erfindung ist daher auch ein Verfahren zum Färben und Tönen von keratischen Fasern, insbesondere menschlischen Haaren, bei dem man ein erfindungsgemäßes Mittel auf das Haar aufbringt, dort für eine Zeitdauer von etwa 1 Minute bis 1 Stunde beläßt und anschließend abspült.

Die nachfolgenden Beispiele sollen den Erfindungsgegenstand näher erläutern.

### Ausführunesbeispiele

Alle Mengenangaben in den Beispielen sind Gewichtsteile.

| 1. Färbelotion | |
|---|---|
| C₁₂₋₁₈-Fettalkohol | 3,00 |
| Emulgade ®1000 NI¹ | 2,00 |
| Laurinsäure | 3,00 |
| Texapon®N 70² | 3,00 |
| Dehyton®K³ | 3,00 |
| Paridol®M⁴ | 0,20 |
| Paridol®P⁵ | 0,20 |
| Isoascorbinsäure | 0,10 |
| Trilon®B⁶ | 0,25 |
| 2-Amino-2-methylpropanol | 0,70 |
| HC Blue 2⁷ | 0,70 |
| Violet 1,4 D⁸ | 0,30 |
| HC Yellow 2⁹ | 0,40 |
| Duftstoffe | 0,30 |
| Wasser dest. | ad 100 |

| | |
|---|---|
| ¹ nichtionogenes Cetylstearylalkohol-Emulgator-Gemisch (INCI-Bezeichnung: Cetearyl Alkohol (and) Ceteareth-20) (HENKEL) | |
| ² Natriumlaurylethersulfat (ca. 72 % Aktivsubstanz in Wasser; INCI-Bezeichnung: Sodium Laureth Sulfate) (HENKEL) | |
| ³ Fettsäureamid-Derivat mit Betainstruktur der Formel R-CONH-(CH₂)₃-N⁺(CH₃)₂CH₂COO⁻ (ca. 30 % Aktivsubstanz in Wasser; INCI-Bezeichnung: Cocamidopropyl Betaine) (HENKEL) | |
| ⁴ 4-Hydroxybenzoesäuremethylester (INCI-Bezeichnung: Methylparaben) (NAARDEN) | |
| ⁵ 4-Hydroxybenzoesäurepropylester (INCI-Bezeichnung: Propylparaben) (NAARDEN) | |
| ⁶ Ethylendiamintetraessigsäure-tetra-Natrium-Salz (40 % Aktivsubstanz in Wasser, INCI-Bezeichnung: Tetrasodium-EDTA) (BASF) | |
| ⁷ N,N,N'-Tris(2-hydroxyethyl)-2-nitro-1,4-phenylendiamin (GRAFOX) | |
| ⁸ N,N'-Bis(2-hydroxyethyl)2-nitro-1,4-phenylendiamin (GRAFOX) | |
| ⁹ 1(2-Hydroxyethylamino)-2-nitrobenzol (GRAFOX) | |

Die Färbelotion hatte einen pH-Wert von 7.

| 2. Färbelotion | |
|---|---|
| C₁₂₋₁₈-Fettalkohol | 3,00 |
| Emulgade ®1000 NI | 2,00 |
| Laurinsäure | 3,00 |
| Texapon®N 70 | 3,00 |
| Dehyton®K | 3,00 |
| Lutrol®E 1500¹⁰ | 1,00 |
| Paridol®M | 0,20 |
| Paridol®P | 0,20 |
| Isoascorbinsäure | 0,10 |
| Trilon®B | 0,25 |
| 2-Amino-2-methylpropanol | 0,70 |
| HC Blue 2 | 1,50 |
| HC Red 3¹¹ | 0,10 |
| Rodol 9 R¹² | 0,10 |
| HC Yellow 2 | 0,15 |
| Diethylenglykolmonoethylether | 3,00 |
| Duftstoffe | 0,30 |
| Wasser dest. | ad 100 |

| | |
|---|---|
| ¹⁰ Polyethylenglykol (INCI-Bezeichnung: PEG 32) (BASF) | |
| ¹¹ N(2-Hydroxyethyl)-2-nitro-1,4-phenylendiamin (GRAFOX) | |
| ¹² 6-Chlor-4-nitro-2-aminophenol (LOWENSTEIN) | |

Die Färbelotion hatte einen pH-Wert von 7.

## Patentansprüche

1. Mittel zum Färben oder Tönen keratinischer Fasern, insbesondere menschlichen Haaren, **dadurch gekennzeichnet**, daß es neben einem direktziehenden Farbstoff eine Wirkstoffkombination, bestehend aus
- einer Aminopolycarbonsäure oder deren Salz mit einem physiologisch verträglichen Kation,
- Isoascorbinsäure oder deren Salz mit einem physiologisch verträglichen Kation und
- eine gesättigte Carbonsäure mit 10 - 18 Kohlenstoffatomen
enthält.

2. Mittel nach Anspruch 1, **dadurch gekennzeichnet**, daß die Aminopolycarbonsäure ausgewählt ist aus Nitrilotriessigsäure, Ethylendiamintetraessigsäure, Diethylentriaminopentaessigsäure und N-Hydroxyethylethylendiaminotriessigsäure.

3. Mittel nach Anspruch 2, **dadurch gekennzeichnet**, daß die Aminopolycarbonsäure Ethylendiamintetraessigsäure ist.

4. Mittel nach einem der Ansprüche 1 bis 3, **dadurch gekennzeichnet**, daß es sich bei dem Salz um ein Alkali-, Erdalkali-, Aluminium-, Ammonium- oder Mo-no-, Di- oder Trialkanolammoniumsalz handelt.

5. Mittel nach Anspruch 4, **dadurch gekennzeichnet**, daß es sich um das Di- oder Tetranatriumsalz der Ethylendiamintetraessigsäure handelt.

6. Mittel nach einem der Ansprüche 1 bis 4, **dadurch gekennzeichnet**, daß die Aminopolycarbonsäure oder ihr Salz in einer Menge von 0,01 bis 1 Gew.-%, bezogen auf das gesamte Mittel, enthalten ist.

7. Mittel nach einem der Ansprüche 1 bis 6, **dadurch gekennzeichnet**, daß die Isoascorbinsäure in einer Menge von 0,01 - 1 Gew.-%, bezogen auf das gesamte Mittel, enthalten ist.

8. Mittel nach einem der Ansprüche 1 bis 7, **dadurch gekennzeichnet**, daß es sich um Laurinsäure handelt.

9. Mittel nach einem der Ansprüche 1 bis 8, **dadurch gekennzeichnet**, daß es weiterhin ein Polyethylenglykol, ein Polypropylenglykol oder ein Polyglycerin mit einer Molmasse von 500 bis 3000 Dalton enthält.

10. Mittel nach Anspruch 9, **dadurch gekennzeichnet**, daß es ein Polyethylenglykol mit einer Molmasse zwischen 1000 und 2000 Dalton enthält.

11. Verwendung eines Mittels nach einem der Ansprüche 1 bis 10 zum Färben und Tönen von keratinischen Fasern, insbesondere menschlichen Haaren.

12. Verfahren zum Färben und Tönen von keratischen Fasern, insbesondere menschlischen Haaren, **dadurch gekennzeichnet**, daß man ein Mittel nach einem der Ansprüche 1 bis 10 auf das Haar aufbringt, dort für eine Zeitdauer von etwa 1 Minute bis 1 Stunde beläßt und anschließend abspült.

## Claims

1. A formulation for coloring or tinting keratin fibers, more particularly human hair, **characterized in that**, in addition to a substantive dye, it contains an active-substance combination consisting of
- an aminopolycarboxylic acid or salt thereof with a physiologically compatible cation,
- isoascorbic acid or a salt thereof with a physiologically compatible cation and
- a saturated carboxylic acid containing 10 to 18 carbon atoms.

2. A formulation as claimed in claim 1, **characterized in that** the aminopolycarboxylic acid is selected from nitrilotriacetic acid, ethylenediamine tetraacetic acid, diethylenetriamine pentaacetic acid and N-hydroxyethyl ethylenediamine triacetic acid.

3. A formulation as claimed in claim 2, **characterized in that** the aminopolycarboxlic acid is ethylenediamine tetraacetic acid.

4. A formulation as claimed in any of claims 1 to 3, **characterized in that** the salt is an alkali metal, alkaline earth metal, aluminium, ammonium or mono-, di- or trialkanolammonium salt.

5. A formulation as claimed in claim 4, **characterized in that** the salt is the di- or tetrasodium salt of ethylenediamine tetraacetic acid.

6. A formulation as claimed in any of claims 1 to 4, **characterized in that** the aminopolycarboxylic acid or its salt is present in a quantity of 0.01 to 1% by weight, based on the formulation as a whole.

7. A formulation as claimed in any of claims 1 to 6, **characterized in that** the isoascorbic acid is present in a quantity of 0.01 to 1% by weight, based on the formulation as a whole.

8. A formulation as claimed in any of claims 1 to 7, **characterized in that** the carboxylic acid is lauric acid.

9. A formulation as claimed in any of claims 1 to 8, **characterized in that** it additionally contains a polyethylene glycol, a polypropylene glycol or a polyglycerol with a molecular weight of 500 to 3,000 dalton.

10. A formulation as claimed in claim 9, **characterized in that** it contains a polyethylene glycol with a molecular weight of 1,000 to 2,000 dalton.

11. The use of the formulation claimed in any of claims 1 to 10 for coloring and tinting keratin fibers, more particularly human hair.

12. A process for coloring and tinting keratin fibers, more particularly human hair, **characterized in that** the formulation claimed in any of claims 1 to 10 is applied to the hair, left thereon for about 1 minute to about 1 hour and then rinsed off.

## Revendications

1. Agent pour colorer ou teinter des fibres kératiniques, en particulier les cheveux humains, **caractérisé en ce qu**'il renferme outre un colorant montant directement sur la fibre, une association de substances actives constituée
- d'un acide aminopolycarboxylique ou d'un sel de celui-ci avec un cation physiologiquement tolérable,
- d'acide isoascorbique ou d'un sel de celui-ci avec un cation physiologiquement tolérable et
- d'un acide carboxylique saturé comportant 10 à 18 atomes de carbone.

2. Agent selon la revendication 1, **caractérisé en ce que** l'acide aminopolycarboxylique est sélectionné parmi les acides nitrilotriacétique, éthylènediaminotétraacétique, diéthylènetriaminopentaacétique et N-hydroxyéthyléthylènediaminotriacétique.

3. Agent selon la revendication 2, **caractérisé en ce que** l'acide aminopolycarboxylique est l'acide éthylènediaminotétraacétique.

4. Agent selon une des revendications 1 à 3, **caractérisé en ce que** le sel est un sel de métal alcalin, de métal alcalino-terreux, d'aluminium, d'ammonium ou de mono-, de di- ou de trialcanolammonium.

5. Agent selon la revendication 4, **caractérisé en ce qu**'il s'agit du sel di- ou tétrasodique de l'acide éthylènediaminotétraacétique.

6. Agent selon une des revendications 1 à 4, **caractérisé en ce que** l'acide aminopolycarboxylique ou le sel de celui-ci est contenu dans une proportion de 0,01 à 1 % en poids, par rapport à la totalité de l'agent.

7. Agent selon une des revendications 1 à 6, **caractérisé en ce que** l'acide isoascorbique est contenu dans une proportion de 0,01 à 1 % en poids, par rapport à la totalité de l'agent.

8. Agent selon une des revendications 1 à 7, **caractérisé en ce qu**'il s'agit d'acide laurique.

9. Agent selon une des revendications 1 à 8, **caractérisé en ce qu**'il contient en outre un polyéthylèneglycol, un polypropylèneglycol ou une polyglycérine présentant une masse molaire de 500 à 3000 Dalton.

10. Agent selon la revendication 9, **caractérisé en ce qu**'il contient un polyéthylèneglycol présentant une masse molaire comprise entre 1000 et 2000 Dalton.

11. Utilisation d'un agent selon une des revendications 1 à 10 pour colorer et teinter des fibres kératiniques, en particulier les cheveux humains.

12. Procédé pour colorer et teinter des fibres kératiniques, en particulier les cheveux humains, **caractérisé en ce que** l'on applique un agent selon une des revendications 1 à 10 sur la chevelure, on y laisse ledit agent pendant une durée d'environ 1 minute à 1 heure et on le rince ensuite.
